# EUROPEAN PATENT SPECIFICATION

(11) **EP 0 972 519 B1**
(45) Date of publication and mention of the grant of the patent: **16.08.2006**
(21) Application number: 98905773.2
(22) Date of filing: 04.03.1998
(51) Int. Cl.: A61K 35/28, A61K 35/14

(54) **IMMUNE TOLERANCE INDUCERS**
INDUKTOREN FÜR IMMUNTOLERANZ
INDUCTEURS DE TOLERANCE IMMUNITAIRE

(30) Priority: 07.03.1997 JP 5293097; 12.06.1997 JP 15501597; 16.12.1997 JP 34673797
(43) Date of publication of application: 19.01.2000
(73) Proprietor: Jimro Co., Ltd., Takasaki-shi, Gunma 370-0021 (JP)
(72) Inventor: JIN, Tienan, Osaka-shi Osaka 536-0012 (JP); SUGIURA, Kikuya, Hirakata-shi Osaka 573-1155 (JP); MORITA, Haruo, Osaka-shi Osaka 535-0003 (JP); IKEHARA, Susumu, Osaka-shi Osaka 534-0016 (JP); SOGO, Shinji, Tokushima-shi Tokushima 770-0943 (JP); YAMANISHI, Kazuya, Tokushima-shi Tokushima 770-8012 (JP); ADACHI, Masakazu, Takasaki-shi Gunma 370-0864 (JP)
(74) Representative: HOFFMANN EITLE
(86) International application number: PCT/JP1998/000909
(87) International publication number: WO 1998/039016

(56) References cited:
- JP-A- 6 298 654
- JP-T- 60 502 103
- EID A ET AL: "Induction of transplantation tolerance by intraportal injection of allogeneic bone marrow cells. Possible implications for intrauterine bone marrow transplantation across major histocompatibility barriers." TRANSPLANT INTERNATIONAL, (1988 JUL) 1 (2) 109-12. , XP000872310
- GORCZYNSKI R M ET AL: "Prolongation of rat small bowel or renal allograft survival by pretransplant transfusion and/or by varying the route of allograft venous drainage." TRANSPLANTATION, (1994 OCT 15) 58 (7) 816-20. , XP000872326
- YOSHIMURA N ET AL: "The effects of perioperative portal venous inoculation with donor lymphocytes on renal allograft survival in the rat. I. Specific prolongation of donor grafts and suppressor factor in the serum." TRANSPLANTATION, (1990 JAN) 49 (1) 167-71. , XP000872316
- MORITA, HARUO ET AL: "A strategy for organ allografts without using immunosuppressants or irradiation" PROC. NATL. ACAD. SCI. U. S. A. (1998), 95(12), 6947-6952 , XP000872835
- ZHANG Y. et al.: EUR. J. IMMUNOL., 1994, Vol. 24, No. 7, pages 1558-1565, XP002914409

## Description

### BACKGROUND ART

The immunosuppressant is an indispensable adjunct to organ transplantation and novel immunosuppressants are being developed one after another. According to the intended application (use), immunosuppressants can be classified into two categories. Drugs of one category are those which are taken daily as long as the graft remains in the recipient's body for the prophylactic suppression of graft rejection and are variously called maintenance immunosuppressants, prophylactic immunosuppressants, and basal immunosuppressants. Drugs of the other category are those used in massive doses, though for limited periods of time, with an aim to causing an intensive immunosuppression necessary for the cure of the rejection response which may occur notwithstanding sustained immunosuppression, chiefly cellular rejection, and are known as therapeutic drugs for graft rejection.

However, in terms of the principal pharmacologic action as well as side effects, those immunosuppressants can hardly be considered harmless to the human body, and since long-term maintenance doses and/or high doses are required, toxic effects and/or adverse drug reactions which cannot be disregarded are inevitable. Furthermore, those immunosuppressants, when used independently, are not potent enough to produce sufficient immunosuppression, nor are they capable of curing graft rejections after onset at a high cure rate.

Meanwhile, there are clinical reports, though sporadically, on the success with which grafts were maintained even without administration of an immunosuppressant, and those favorable outcomes have been attributed to the induction of immunological tolerance. If such immunotolerance could be actually established, administration of immunosuppressants would not be necessary. Therefore, the artificial induction of immunological tolerance is regarded as a supreme objective in organ transplantation today and results of many relevant studies have been reported.

On the methodology for artificial induction of immunological tolerance, reference may be made to the following report, among others.

Induction of Tolerance by Transfer of a Splenocyte or Myelocyte Tolerogen in Combination with Administration of an Antimitotic Drug [Fukuoka Acta Med., 81(1), 20-40 (1990); Microbiol. Immunol., 32(3), 283-292 (1988), etc.].

As the antimitotic drug, 6-mercaptopurine, methotrexate, cyclophosphamide (CP), 5-fluorouracil, azathioprine (AZP) and procarbazine are mentioned, and it is warned that cyclosporin A (CsA) and steroids, which are remote from those antimitotic agents in the mode of action, are not suitable for the induction of immunological tolerance.

Hayakawa et al. reported their attempt to induce a donor-specific immunocompromised state with FK506 [Keio Medicine, 72(3), 163-176 (1995)]. Similarly Muramatsu et al. reported on the possibility of inducing immunological tolerance with 15-DSG [Abstract of Papers read before the 20th Congress of the Japan Society of Microsurgery, 89-90 (1994)].

The present inventors reported previously that, in mice, administration of bone marrow cells (particularly hematopoietic stem cells) into the portal vein or by the usual intravenous route results in entrapment of the donor-derived cells in the recipient's liver, establishment of chimerism and induction of immunotolerance [Eur. J. Immunol., 24, 1558 (1994)].

In Eid et al. the induction of transplantation tolerance by intraportal injection of allogeneic bone marrow cells is described as well as possible implications for intrauterine bone marrow transplantation across major histocompatibility barriers [Transplant International, July 1988 1(2) : 109-112]. Radiation was conducted at 400 rad WBI which corresponds to 4 Gy.

The object of this invention is to provide a technology by which the necessary immunological tolerance for organ transplantation can be successfully established. Stated differently, this invention has for its object to provide a novel method for ensuring a positive sustenance of grafts without use of immunosuppressants for maintenance (by long-term administration of an immunosuppressant) and, hence, without risks for serious side effects.

After an intensive study the inventors found that the use described hereinafter meets the above object and have perfected this instant invention.

### DISCLOSURE OF THE INVENTION

The invention provides for inducing immunological tolerance in a patient undergoing an organ transplantation the use of an effective amount of a tolerogen containing hematopoietic stem cells, hematopoietic progenitor cells, mature lymphocytes or a mixture thereof other than derived from a human embryo, in combination with a pharmaceutical carrier for the preparation of a pharmaceutical composition for portal administration for inducing immunological tolerance in a patient undergoing an organ transplantation, in association with total body irradiation using a sublethal radiation dose of at least 6.5 Gy.

Thereby, the present invention provides for the used of an immunotolerance inducer for application to a patient undergoing an organ transplantation in association with radiation for inducing immunological tolerance in said patient comprising an effective amount of a tolerogen containing hematopoietic stem cells, hematopoietic progenitor cells or a mixture thereof in combination with a pharmaceutical carrier, more particularly said immunotolerance inducer comprising a bone marrow cell fraction as said tolerogen.

By using the immunotolerance inducer of this invention, the immunological tolerance meeting the above-mentioned object can be successfully established so that the transplanted organ can be maintained in satisfactory condition.

As far as this constitution is retained, the pharmaceutical artefact and dosage form of each composition are not particularly restricted.

For example, said pharmaceutical compositions may be provided in a single dosage form or optionally in independent dosage forms. Thus, as typically represented by the examples of use given hereinafter for the immunotolerance inducer of this invention, there is no particular limitation on the type of pharmaceutical artefact and dosage form only provided that the object of inducing the necessary immunological tolerance is accomplished.

The tolerogen containing hematopoietic stem cells, hematopoietic progenitor cells, mature lymphocytes or a mixture thereof, which is the active ingredient in said pharmaceutical compositions in common may for example be a tolerogen derived from the graft donor (an animal of the same strain as the donor). The active ingredient mentioned above may be a bone marrow cell fraction, spleen cell fraction, peripheral blood cell fraction or a fraction comprising a mixture of them, which contains said cells.

The separation and isolation of such tolerogens can be carried out by known procedures. For example, the procedure described by Yamamoto et al. [Blood, 88, 445-454 (1996) and the procedure described in Protocols in Experimental Cellular Immunity [ed. by Mishell B. B., Shiigi S. M.; translated by Katsuyuki Imai, Susumu Kawaguchi and Takayuki Harada, Rikogaku-Sha, pp.3-12, 1982] can be employed.

The preferred tolerogen from the graft donor (a human) includes bone marrow cells and peripheral blood cells. The method of harvesting those cells is well known to those skilled in the art. For example, the method for handling bone marrow cells may be the same as that used in bone marrow transplantation.

The tolerogen for use in the pharmaceutical composition is preferably a bone marrow cell fraction rich in hematopoietic progenitor cells, a spleen cell or peripheral blood cell fraction containing mature lymphocytes (exclusive of activated lymphocytes) or a mixture thereof. On the other hand, the tolerogen for the pharmaceutical composition is preferably said bone marrow cell fraction. As the active component of said pharmaceutical compositions, a bone marrow cell fraction is preferred as mentioned above but the peripheral blood cell fraction containing the hematopoietic stem cells mobilized from the bone marrow by cytokines such as G-CSF is also preferred partly because it contains both of mature lymphocytes and hematopoietic progenitor cells and partly because such a cell fraction is readily available.

To provide said pharmaceutical compositions, the active component can be formulated into a conventional dosage form known for pharmaceutical products containing cellular fractions of this type. The dosage form can be judiciously selected from among a variety of dosage forms for the present purpose. An injectable dosage form can be mentioned as an example. The pharmaceutical carrier or vehicle which can be used in the manufacture of such dosage forms includes a broad range of pharmaceutically acceptable substances. The method of preparation may also follow the established pharmaceutical procedures. In preparing such dosage forms, the various infusions which are in broad use nowadays can also be employed.

In the practice of this invention, said dosage forms can be prepared extemporaneously, if desired, on the occasion of organ transplantation, using the material obtained from the graft donor.

The dosage and timing of administration of each pharmaceutical composition are not particularly restricted but can be judiciously elected by those skilled in the art only provided that the necessary immunological tolerance may be successfully established.

The recommended dosage of the pharmaceutical composition is the minimum dose (3x10⁷ cells in mice) required to ensure that said reactivity to the donor's alloantigen in the mixed lymphocyte reaction after administration becomes minimal (maximum inhibition of the reaction) and plateau out.

The mixed lymphocyte reaction test for spleen cells, referred to above, can be carried out in the routine manner [Protocols in Experimental Cellular Immunity, 147-149, 1982]. The total dosage for administration in humans may be the dose used in the conventional bone marrow transplantation. For example, the dosage in terms of bone marrow cells may be about 3x10⁸ cells/kg or more.

This invention, therefore, provides a method of inducing immunological tolerance using the specific procedures described above.

The above-mentioned result achieved by the method of this invention is not related to the timing of transplantation of a graft material. Thus, the transplantation procedure can be successfully carried out, whether in parallel with the procedure of this invention or after the establishment of immunological tolerance by the procedure of this invention.

In inducing immunological tolerance in accordance with this invention, various other medical treatments and administration of drugs, which are concomitantly practiced in procedures of this kind can be practiced in combination with the procedure of the invention unless the effect of the invention is thereby compromized.

As an example, administration of said immunosuppressants can be mentioned. The method, dosage, and timing of administration of immunosuppressants can be judiciously selected by those skilled in the art.

The particularly preferred immunosuppressant includes cyclosporin A and FK506, to mention just a few representative drugs, and the dosage and administration method may be those recommended for the known commercial products. The particularly preferred mode of administration is to administer an immunosuppressant shortly after administration of the pharmaceutical composition, once or twice, for example around the 2nd day or around the 2nd and 5th days following portal administration.

Furthermore, the present invention provides an immunotolerance inducing technology capable of introducing chimerism with a minimum of invasion and with a high degree of certainty to ensure a long-term sustenance of the state of immunological tolerance.

Thus, the present invention provides an immunotolerance inducer to be applied, in association with radiation, to a patient undergoing an organ transplantation for inducing immunological tolerance in the patient, which comprises an effective amount of a tolerogen containing hematopoietic stem cells, hematopoietic progenitor cells or a mixture thereof and a pharmaceutically acceptable carrier.

The immunotolerance meeting the object mentioned hereinbefore can be established as far as the procedure is followed in combination with radiation, with the result that the transplanted organ can be maintained in satisfactory condition.

The tolerogen containing hematopoietic stem cells, hematopoietic progenitor cells or a mixture thereof which constitutes the active component of the medicine of this invention for administration to the patient in association with radiation, may for example be a bone marrow cell fraction, a peripheral blood cell fraction, or a mixture thereof, which contains hematopoietic stem cells and hematopoietic progenitor cells.

The tolerogen derived from the graft donor (a human) includes a bone marrow cell fraction, an umbilical blood cell fraction and a peripheral blood cell fraction containing hematopoietic stem cells mobilized by a cytokine such as G-CSF.

The technique for separation and isolation of said tolerogen for use in the immunotolerance inducer of this invention to be applied in association with said radiation, the method of manufacture of the inducer, its dosage form, the pharmaceutical carrier for use in the manufacture of the dosage form, the route of administration and dosage are the same as those mentioned hereinbefore for the above pharmaceutical composition.

It is essential, however, that the above immunotolerance inducer of this invention be used in association with radiation, that is to say it be administered into the portal vein to the patient given said radiation.

The reference dosage for intravenous administration is roughly the dose (3x10⁷ cells in mice) required for reconstructing the host's immune system in the transplantation of the ordinary major histocompatibility complex (MHC)-incompatible bone marrow (after irradiation in a lethal dose in mice).

With the above dosage for mice being taken as a reference, the dosage of the medicine of this invention can be judiciously selected according to the conventions of bone marrow transplantation. As a specific example, the dose of about 3x10⁸ cells/kg or more, in terms of bone marrow cells, can be mentioned.

The radiation mentioned above can be carried out in the conventional manner. More particularly, the patient (recipient) undergoing an organ transplantation is exposed to a suitable radiation dose, of at least 6.5 Gy and yet sublethal dose, preferably about 7.0 Gy, per exposure, on a total body irradiation (TBI) basis. This radiation dose is characterized also as a radiation dose providing for recovery of the recipient's bone marrow cells.

The above radiation can be carried out before administration of the medicine of this invention and usually the medicine is preferably administered within 24 hours of irradiation.

This invention is advantageous in that the expected efficacy can be obtained by a single dose of medication which is least invasive to the recipient.

By administering the medicine of this invention in conjunction with radiation, the desired immunological tolerance can be induced for a satisfactory maintenance of the transplanted organ.

The present invention, therefore, provides an immunotolerance inducing method involving radiation.

The phenomenon that the desired immunological tolerance is induced by this method involving radiation for a successful maintenance of the graft is also unrelated to the timing of the operation for transplantation of the graft.

In practicing the above combination treatment method, too, the various medical treatments and medications which are usually given in procedures of this kind, for example administration of immunosuppressive drugs such as cyclosporin A, FK506, etc., can be carried out concomitantly unless the effect of the invention is diminished or cancelled.

### BRIEF DESCRIPTION OF THE DRAWINGS

Fig. 1 is a diagrammatic repre sentation of the engraftment rate of skin grafts in Test Example 3 and
Fig. 2 is a diagrammatic representation of the engraftment rate of skin grafts in Test Example 4.

### BEST MODE FOR CARRYING OUT THE INVENTION

The following is a description of the tests performed with the active component of this invention for illustrating this invention in further detail.

### Test Example 1 (reference only)

The induction of immunological tolerance in the test examples was effected by (1) the injection of an allogeneic donor's spleen cells or bone marrow cells into the portal vein and (2) the intravenous injection of the allogeneic donor's bone marrow cells and the establishment of immunological tolerance was evaluated using the engraftment rate of skin grafts (allogeneic to the donor) which is the organ most susceptible to rejection as an indicator.

### (1) Preparation of a spleen cell suspension

Spleen cells were harvested from 8-week-old female BALB/cCrSlc mice (body weights 19-22 g, BALB/c; Japan SLC Inc.) and loosened up with a pair of non-toothed forceps on a 200 G stainless steel mesh screen in RPMI1640 solution (Nikken Bio Med. Lab.) to prepare discrete spleen cells. The cells were washed with RPMI1640 solution once and subjected to hemolysis with Tris-HCl-ammonium chloride buffer (0.75% NH₄Cl, 0.017 M Tris-HCl, pH 7.5). After two further washings with RPMI1640, the spleen cells were resuspended in the same solution to provide a spleen cell suspension (concentration: 1.0x10⁸/ml).

### (2) Preparation of a bone marrow cell suspension

The femurs and tibias were isolated from 8-week-old female BALB/c mice and a 22-G needle (Code No. NN-2225R, Terumo Co., Ltd.) attached to a syringe (2.5 ml, Code No. SS-02S, Terumo Co., Ltd.) was inserted into each bone from the knee joint side and the bone marrow cells were flushed into a sterilized dish (90x16 mm, Iwaki Clinical Test Ware) using RPMI1640 solution and suspended in RPMI1640 solution. The bone marrow cells thus harvested were washed with RPMI1640 solution once and resuspended in the same solution to provide the objective bone marrow cell suspension (concentration: l.Oxl0 /ml).

### (3) Injection into the portal vein

Under pentobarbital anesthesia ( Pitman-Moor Inc.; 37.5 mg/kg body weight, i.p.), 10-week-old female C57BL/6CrSlc mice (B6; body weights 20-24 g, Japan SLC Inc.) were shaved of hairs with a razor and disinfected. Then, a midline incision was made in the abdominal region and the mesenterium was exposed. A 27 G needle (Terumo Co., Ltd.) attached to a 1 ml-tuberculin syringe was inserted through the adipose tissue of the mesenterium and 3x10⁷ BALB/c mouse spleen cells or bone marrow cells (0.3 ml suspension) prepared in (1) above were administered into the portal vein.

### (4) Intravenous injection

The bone marrow cell suspension prepared in (1) above was adjusted to a concentration of 1x10⁸ cells/ml and the 3x10⁷ cells equivalent of the suspension (0.3 ml) was administered into the tail vein of the host mouse at day 5 after the portal injection described in (3) above.

### (5) Skin grafting

Skin grafting was carried out at day 7 after portal injection. The preparation of skin graft materials and the transplantation thereof were carried out as follows, with reference to the procedure described in the literature [Mayumi et al., Jpn. J. Surg., 18, 548-557 (1988)].

Thus, as the donor, 8-week-old BALB/c mice were sacrificed under ethyl ether ( Nacalai Tesque Inc.) anesthesia. Using a depilatory cream (Feather Hair Remover, Feather Softy Razor Co., Ltd.), the whole hair coat was removed and after disinfection with 70% alcohol, the full-thickness skin layer was peeled off and recovered. After the subcutaneous adipose tissue was removed with a pair of forceps (bent tip, tapered, non-toothed) and sanitary cotton balls as much as possible, the skin was cut into a flap (1.2x1.5 cm²). A 1 mm-long incision was made on the cranial side of the flap as a marker and the flap was suspended in cold sterile phosphate-buffered saline (Dulbecco's PBS(-), Nissui Pharmaceutical Co., Ltd.).

After a B6 host mouse was anesthetized with pentobarbital (37.5 mg/kg body weight, i.p.), the right dorsal region was plucked of hairs with fingers and further depilated with said depilatory cream (3.0x3.5 cm) and disinfected with 70% alcohol to prepare an operating field for skin grafting.

On the denuded area, the BALB/c skin flap prepared above was placed with the marker disposed caudad and sutured in 8 stitches (in the center of each side and at the 4 corners) using a nylon suture with a 6-0 needle (Ethilon; Ethicon Inc.). The surface of the skin graft was covered with a patch of gauze carrying a fradiomycin sulfate ointment (2.0x2.5 cm, Sofratulle; Japan Roussel Co., Ltd.) and further occluded with an adhesive elastic bandage (Elatex; Alcare Co., Ltd.).

The check for engraftment was started at week 2 after transplantation.

### (6) Results

The results are shown in Table 1.

**Table 1**

| | Tolerance procedure | | Engraftment of skin graft | |
|---|---|---|---|---|
| | p.v. | i.v | Time after grafting | Engraftment rate (%) |
| Test group 1 | Spleen Cells | Marrow Cells | 36 | 100 (10/10) |
| Test group 2 | Spleen Cells | Spleen Cells | 18 | 20 (1/5) |
| Test group 3 | Marrow Cells | Marrow Cells | 36 | 67 (4/6) |
| Control group 1 | Spleen Cells | - | 3 | 0 (0/4) |
| Control group 2 | Marrow Cells | - | 3 | 0 (0/4) |

### (7) Explanation of results and discussion

Test group 1: BALB/c mouse-derived spleen cells were administered into the portal vein of 10 MHC-incompatible B6 mice. At day 5 after administration, BALB/c mouse bone marrow cells were injected intravenously, and at day 7, skin grafting was performed. As a result, engraftment of the transferred skin material was confirmed in 10 of 10 mice at week 36 after transplantation.

Test group 2: BALB/c mouse-derived spleen cells were administered into the portal vein of 5 B6 mice and at day 5 after administration, BALB/c mouse-derived spleen cells were injected intravenously. Skin grafting was performed at day 7. As a result, engraftment was confirmed in 1 of 5 mice at week 18 after transplantation but the graft was rejected (dislodged) in one mouse at week 6 and in 3 mice at week 7.

Test group 3: BALB/c mouse bone marrow cells were administered into the portal vein of 6 B6 mice, and at day 5, BALB/c mouse-derived bone marrow cells were administered intravenously. Skin grafting was performed at day 7. As a result, engraftment of the transferred skin was found in 4 of 6 mice at week 36 after transplantation. Control group: BALB/c mouse-derived spleen cells were administered into the portal vein of 4 B6 mice and skin grafting was performed at day 7. As a result, the graft was rejected (dislodged) in 2 mice at week 2 after transplantation and in the remaining 2 mice at week 3. Control group 2: BALB/c mouse-derived bone marrow cells were administered into the portal vein of 4 B6 mice and skin grafting was performed at day 7. As a result, the skin graft was rejected in 2 mice at week 2 after transplantation and in the remaining 2 mice at week 3.

It is clear from the above results that the portal administration of the first pharmaceutical composition and subsequent intravenous administration of the second pharmaceutical composition ensure a successful engraftment of the donor's skin graft (maintenance of the donor's alloantigen-specific immunotolerance).

Furthermore, when an immunosuppressant was administered between the portal administration (day 0) and intravenous administration (day 5) of bone marrow cells in the above test group 3, an improvement was obtained in the engraftment rate of transferred skin grafts. The following test examples will cast more light on the above findings.

### Test Example 2 (reference only)

### (1) Preparation of bone marrow cells and administration into the portal vein and by the intravenous route

Bone marrow cells were prepared and administered in the same manner as the above Test Example 1-(2), (3) and (4).

### (2) Administration of an immunosuppressant

As the immunosuppressant, either cyclosporin A (CsA; Sandimmum, 250 mg/5 ml solution, Novartis Pharma K.K.) 10 mg/kg body weight or FK506 (10 mg/ml solution, Fujisawa Pharmaceutical Co., Ltd.) 1 mg/kg body weight was administered intraperitoneally at day 2 and day 5 after portal administration.

### (3) Skin grafting

Skin grafting was performed in the same manner as Test Example 1-(5).

### (4) Results

The results are shown below in Table 2.

**Table 2**

| | Tolerance procedure | | | Engraftment of skin graft | |
|---|---|---|---|---|---|
| | p.v. | Immunosupprresant | i.v | Time (W) after grafting | Engraftment rate (%) |
| Test group 3 | Marrow Cells | - | Marrow Cells | 36 | 67 (4/6) |
| Test group 4 | Marrow Cells | CsA | Marrow Cells | 32 | 80 (4/5) |
| Test group 5 | Marrow Cells | FK506 | Marrow cells | 30 | 83 (5/6) |
| Control group 2 | Marrow Cells | - | | 3 | 0 (0/4) |

### (5) Explanation of results and discussion

Test group 3 and control group 2 have been fully described in the section of Test Example 1.

Test group 4: BALB/c mouse-derived bone marrow cells were administered into the portal vein of 5 B6 mice. At day 2 and day 5, CsA was administered. In addition, BALB/c mouse-derived bone marrow cells were administered intravenously at day 5 and skin grafting was performed at day 7. As a result, the skin graft was rejected in 1 mouse at week 6 after transplantation but engraftment was obtained in 4 of 5 mice at week 32 after transplantation.

Test group 5: BALB/c mouse-derived bone marrow cells were administered into the portal vein of 6 B6 mice. At day 2 and day 5, FK506 was administered. In addition, BALB/c mouse-derived bone marrow cells were administered intravenously at day 5 and skin grafting was performed at day 7. As a result, the skin graft was rejected in 1 mouse at week 6 after transplantation but engraftment was obtained in 5 of 6 mice at week 30 after transplantation.

The following conclusion can be drawn from the above findings. Although it was generally acknowledged that immunosuppressants such as CsA and FK506 are not suited for use in combination with a tolerogen for the induction of immunotolerance, the use of an immunosuppressant in combination with the portal administration of the first pharmaceutical composition and the intravenous administration of the second pharmaceutical composition results in an improved engraftment rate and is, therefore, effective in inducing immunological tolerance.

### Test Example 3 (reference only)

### (1) Preparation of bone marrow cells and the portal and intravenous injection of the cells

The procedures described in Example 1-(2), (3) and (4) were repeated.

### (2) Administration of an immunosuppressant

CsA, 10 mg/kg body weight, was administered intraperitoneally at day 2 and day 5 after portal injection.

### (3) Skin grafting

Except that skin grafting was performed on the same day as the portal administration, the procedure of Test Example 1-(5) was repeated (n=6). A control group receiving skin flaps derived from C3H mice was also provided (n=4).

### (4) Results

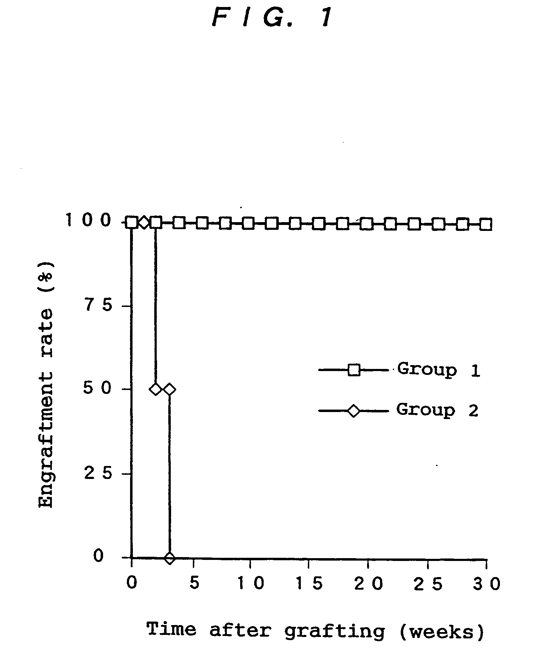

The results are shown in Fig. 1.

In Fig. 1, the ordinate represents the engraftment rate (%) of skin flaps and the abscissa represents time (in weeks) after grafting. Group 1 is a test group and Group 2 is a control group.

The results of this test example indicate that the immunotolerance inducing procedure comprising administration of the first pharmaceutical composition into the portal vein and the organ transplantation can be concurrently carried out. Therefore, in humans, too, the portal administration of the first pharmaceutical composition (bone marrow and other cells) from the donor and the organ transplantation can be concurrently performed. This technique is considered to be an epochal one in that the graft vs. host reaction (GvH reaction) can be prevented even without removal of T cells from the marrow cell fraction and the immunotolerance can be sufficiently maintained using only two doses of an immunosuppressant.

### Test Example 4 (inventive)

The induction of immunotolerance was carried out by the portal or intravenous injection of an allogeneic donor's bone marrow cells and the establishment of immunological tolerance was evaluated using the engraftment rate of the skin grafts ( allogeneic to the donor) which are most susceptible to rejection as an indicator.

### (1) Preparation of a bone marrow cell suspension

From the donor mouse, the femurs and tibias were removed and a 22 G needle (Code No. NN-2225R, Terumo Co., Ltd.) attached to a syringe (2.5 ml, Code No. SS-02S, Terumo Co., Ltd.) was inserted into each bone from the knee-joint side. The bone marrow cells were flushed into a sterilized dish (90x15 mm, Iwaki Clinical Test Ware) using RPMI1640 solution from the syringe and suspended in RPMI1640 solution. The harvested bone marrow cells were washed with RPMI1640 solution once and resuspended in the same solution to provide the objective bone marrow cell suspension (concentration: 1x10⁸ cells/ml).

### (2) Radiation

Irradiation of the recipient mice was carried out by the TBI method using Gamma Cell 40 Exacter (Nordion International Inc.) and ¹³⁷Cs as a beam source.

### (3) Portal administration

The recipient mouse was shaved of hairs with a razor under pentobarbital anesthesia ( Pitman-Moor Inc.; 37.5 mg/kg body weight, i.p.) and after disinfection, a midline incision was made in the abdomen and the mesenterium was exposed. A 27 G needle (Terumo Co., Ltd.) attached to a 1 ml-tuberculin syringe was inserted through the adipose tissue of the mesenterium and 3x10⁷ bone marrow cells from the donor mouse (0.3 ml of the suspension prepared above) were administered into the portal vein.

### (4) Intravenous administration

The bone marrow cell suspension prepared above from the donor mouse was adjusted to 1x10⁸ cells/ml and the 3x10⁷ equivalent thereof (0.3 ml) was administered from the tail vein of the recipient mouse.

### (5) Skin grafting

The preparation and transplantation of skin grafts were carried out as follows, with reference to the procedures described in the literature [Mayumi et al., Jpn. J. Surg., 18, 548-557 (1988)].

Thus, the donor mouse was sacrificed under ethyl ether (Nacalai Tesque Inc.) anesthesia and the whole hair coat was removed with a depilatory cream (Feather Hair Remover, Feather Safety Razor Co. Ltd.). After disinfection with 70% alcohol solution, the full-thickness skin layer was peeled off and recovered. Using a pair of forceps (with a bent tip, tapered, non-toothed) and sanitary cotton balls, the subcutaneous fat tissue was removed as much as possible and the skin was cut into a flap (1.2x1.5 cm square). A 1 mm-incision was made on the cranial side of the flap as a marker and the skin flap was left floating in cold sterilized phosphate-buffered saline (Dulbecco's PBS(-), Nissui Pharmaceutical Co. Ltd.).

After the recipient mouse was anesthetized with pentobarbital (37.5 mg/kg body weight, i.p.), the right dorsal region was plucked of hairs with fingers and further depilated with said depilatory cream (3.0x3.5 cm). The denuded area was disinfected with 70% alcohol solution to prepare an operating field for skin grafting.

On the denuded area, the donor's skin flap prepared above was placed with the marker disposed caudad and sutured in 8 stitches (in the center of each side and at the 4 corners) using a nylon suture with a 6-0 needle (Ethilon; Ethicon Inc.). The surface of the graft was covered with a patch of gauze carrying a fradiomycin ointment (2.0x2.5 cm, Sofratulle; Japan Roussel Co., Ltd.) and further occluded with an adhesive elastic bandage (Elatex; Alcare Co., Ltd.).

### (6) Induction of immunological tolerance

Using (BALB/cxDBA2) F1 mice (H-2K^{d}) (aged 7~8 weeks, 19∼20 g, Japan SLC) as donor mice and B6 mice (H-2K^{b}) (aged 10∼13 weeks, 20∼23 g, Japan SLC) as recipient mice, each recipient animal was irradiated and, after 1 day, the donor's bone marrow cells were administered either into the portal vein or intravenously. Skin grafting was performed within the same day as the portal or intravenous administration of bone marrow cells and the check for engraftment of skin flaps was made starting week 3 after transplantation.

### (7) Results

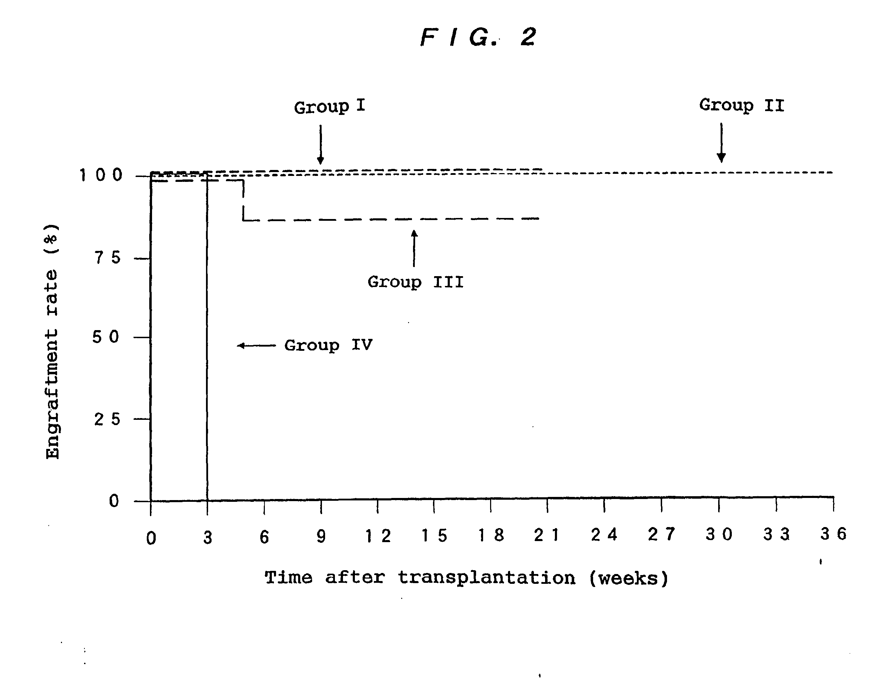

The results are shown in Fig. 2.

In Fig. 2, the ordinate represents engraftment rate (%) and the abscissa represents time (in weeks) after transplantation. The legend Group I represents the data generated in a group (n=3) which received a radiation dose of 6.5 Gy in association with portal administration of bone marrow cells [Group I: 6.5 Gy+pv (n:3)]; the legend Group II represents a group which received a radiation dose of 7.0 Gy in association with portal administration (n=9) or intravenous administration (n=5) of bone marrow cells [Group II: 7 Gy+pv (n=9) or iv (n=5)]; the legend Group III represents a group which received a radiation dose of 6.5 Gy in association with intravenous administration of bone marrow cells (n=7) [Group III: 6.5 Gy+iv (n=7)]; and the legend Group IV represents a group which received a radiation dose of 6.0 Gy in association with portal administration (n=5) or intravenous administration (n=3) of bone marrow cells [Group IV: 6.0 Gy+pv (n=5) or iv (n=3)].

### (8) Explanation of the results

In B6 mice, total body irradiation was performed in a dose of 7.0 Gy, 6.5 Gy or 6.0 Gy and after about 24 hours, the portal (pv) or intravenous (iv) injection of bone marrow cells derived from a (BALB/cxDBA/2) F1 mouse (CDF1) was carried out. Then, within the same day, skin grafting was performed. As shown in Fig. 2, the recipient mice given a radiation dose of 7 Gy in both the portal and intravenous administration groups showed an engraftment rate of 100% for the donor (CDF1)'s skin graft at week 23 (on the 167th day) after transplantation (9 of 9 mice in the pv group and 5 of 5 mice in the iv group). This is in contrast with the recipient mice exposed to a radiation dose of 6.0 Gy, in which the skin graft was invariably rejected within 3 weeks after transplantation (5 of 5 mice in the pv group and 3 of 3 mice in the iv group). In the recipient mice given a radiation dose of 6.5 Gy, the skin graft was rejected in one of 7 mice in the intravenous administration group at week 3 after transplantation but successful engraftment was obtained in 3 of the 3 recipient mice in the portal administration group at week 13 after transplantation.

### (9) Discussion

The engraftment rate was slightly higher in the 6.5 Gy plus portal administration group. It appears that because the donor's hematopoietic stem cells are trapped in the recipient's liver with higher efficiency in this group, the rejection by radio-resistant immunocompetent cells in the recipient mice is more effectively avoided. Pharmaceutical Example 1

Bone marrow cells or spleen cells are suspended in physiological saline to prepare a 1x10⁸/ml composition for administration into the portal vein. On the other hand, a composition containing 1x10⁸ bone marrow cells/ml saline is similarly prepared for intravenous administration.

In humans, the above composition for portal administration is preferably administered in a dose of generally 3x10⁸ bone marrow cells or more (T cells may be present) per kg body weight.

### Pharmaceutical Example 2

Bone marrow cells are suspended in physiological saline to provide a 1x10⁸/ml suspension. For administration into the portal vein of a patient, for instance, the suspension is preferably administered in a dose of generally 3x10⁸ bone marrow cells or more (a small proportion, i.e. about 2%, of T cells may be present) per kg body weight. Thus, there is provided an injection containing at least the above unit dose. This injectable composition is of value as an immunotolerance inducer to be used in association with radiation.

### INDUSTRIAL APPLICABILITY

With the immunotolerance inducer of this invention, an immunological tolerance can be induced in a patient undergoing an organ transplantation and a positive maintenance of the transplanted organ can therefore be ensured.

## Claims

1. Use of an effective amount of a tolerogen containing hematopoietic stem cells, hematopoietic progenitor cells, mature lymphocytes or a mixture thereof, other than those derived from a human embryo, in combination with a pharmaceutical carrier for the preparation of a pharmaceutical composition for portal administration for inducing immunological tolerance in a patient undergoing an organ transplantation, in association with total body irradiation using a sublethal radiation dose of at least 6.5 Gy.

2. The use according to Claim 1, wherein the tolerogen contains bone marrow cells.

## Patentansprüche

1. Verwendung einer effektiven Menge von einem Tolerogen, enthaltend hämatopoietische Stammzellen, hämatopoietische Vorläuferzellen, reife Lymphozyten oder einer Mischung daraus, wobei diese nicht von einem menschlichen Embryo abstammen, in Kombination mit einem pharmazeutischen Träger für die Herstellung einer pharmazeutischen Zusammensetzung für die Pfortaderverabreichung zur Induktion einer immunologischen Toleranz bei einem Patienten, der eine Organtransplantation durchmacht, in Assoziation mit einer Gesamtkörperbestrahlung unter Verwendung einer sublethalen Bestrahlungsdosis von mindestens 6,5 Gy.

2. Verwendung gemäss Anspruch 1, wobei das Tolerogen Knochenmarkszellen enthält.

## Revendications

1. Utilisation d'une quantité effective d'une tolérogène contenant des cellules souches hématopoïétiques multipotentes, des cellules progénitrices hématopoïétiques, des lymphocytes matures ou un mélange de ceux-ci, autres que ceux dérivés d'un embryon humain, en combinaison avec un vecteur pharmaceutique pour la préparation d'une composition pharmaceutique pour l'administration portale afin d'induire une immunotolérance chez un patient subissant une greffe d'organes, en association avec une irradiation totale utilisant une dose d'irradiation sublétale d'au moins 6,5 Gy.

2. Utilisation selon la revendication 1, dans laquelle la tolérogène contient des cellules de la moelle osseuse.
